# EUROPEAN PATENT SPECIFICATION

(11) **EP 0 615 726 B1**
(45) Date of publication and mention of the grant of the patent: **19.01.2000**
(21) Application number: 94250021.6
(22) Date of filing: 04.02.1994
(51) Int. Cl.: A61B 17/06

(54) **Surgical needle**
Chirurgische Nadel
Aiguille chirurgicale

(30) Priority: 13.02.1993 DE 4304739
(43) Date of publication of application: 21.09.1994
(60) Divisional application: 99250233.6
(73) Proprietor: ETHICON INC., Somerville, New Jersey 08876-0151 (US)
(72) Inventor: Brunken, Dieter, D-24641 Hüttblek (DE)
(74) Representative: UEXKÜLL & STOLBERG

(56) References cited:
- EP-A- 0 296 776
- EP-A- 0 564 059
- DE-C- 3 841 443
- DE-C- 4 208 242
- US-A- 3 840 015
- PATENT ABSTRACTS OF JAPAN vol. 13, no. 286 (C-613)[3634] 29 June 1989 & JP-A-01 083 252 (KAIHATSU) 29 March 1989

## Description

The invention relates to a surgical needle with a suture material or thread secured to the end opposite the puncture tip.

Such surgical needles are generally known and in most cases consist of a corrosion-resistant metal, preferably of chrome-nickel steel. With such needles, whose whole length is bare or has not been surface-treated, precise establishment of the puncture point and estimation of the puncture depth is frequently not unproblematical during an operation. In addition, when pulling out the needle after the tissue has been pierced, determination of how much further the needle must still be pulled until its rear section with the thread attachment has also emerged from the tissue is in many cases possible only with difficulty.

Known from U.S. Patent No. 3 840 015 is a surgical needle whose tip is provided with a photoluminescent covering which, when irradiated by a suitable source, is excited to light up. Otherwise the needle is bare or untreated at the surface. The photoluminescent covering improves the visibility of the needle tip, which is a help in establishing the puncture point and greatly facilitates the use of the needle. A disadvantage is that an additional radiation source is necessary to cause the photoluminescent covering to light up. Moreover, such a covering is not unproblematical in terms of its toxicity.

The object of the invention is to provide a surgical needle which can be handled more easily and which makes more precise operation possible.

This object is achieved by a surgical needle with the features of claim 1 and by a surgical needle with the features of claim 2. Advantageous versions result from the dependent claims.

With the inventive surgical needle, the surface of the puncture tip and of the zone of the needle adjoining same is coloured, continuously or with small breaks, by chemical or electrolytic means or by a covering, for up to ca. 50% of the needle length, while the surface of the remaining section of the needle up to the thread attachment consists of bare or untreated metal. As a result of the surface treatment, the zone of the puncture tip stands in clear contrast to the rest of the needle, which can be of great help to the surgeon in the precise establishment of the puncture point. Surprisingly, it turns out that a suture can also be created very precisely with such a needle according to the invention, which differs from the existing view that the needle zone which is to be emphasized must reflect light strongly or even light up itself. The surgical needle according to the invention also provides a marking carrier since, upon insertion of the needle, the surgeon can, by means of the still visible rear section of the surface-treated zone, establish quickly and surely the extent to which the needle has already advanced into the tissue. The operating procedure is greatly facilitated as a result.

In an advantageous version of the surgical needle according to the invention, not only the surface of the puncture tip and of the zone of the needle adjoining same up to ca. 50% of the needle length, but also the surface of the rear section of the needle, which can account for up to ca. 50% of the needle length, up to the thread attachment, are subjected to a surface treatment, while in the middle section of the needle the surface consists of bare or untreated metal. With such a version, both the zone of the puncture tip and the rear section of the needle are clearly marked and contrasted, so that on the one hand the puncture point can be precisely established and on the other the needle is also more recognizable when being pulled out.

The section of the needle subjected to a surface treatment need not be continuously coloured, but can for example also be coloured in annular or speckled form, the surface between the rings or speckles being bare or untreated. The intermediate spaces lying bare in the zone which is coloured with breaks are preferably not to be larger than 1 to 2 mm. This gap is enough to create a closed impression of the zone of the puncture tip or of the rear section of the needle. On the other hand, the bare areas can improve the visibility or conspicuousness of the corresponding needle zone.

The invention will be explained in more detail below with the help of embodiments. The drawings show:
- Figure 1: a magnified representation of a surgical needle according to the invention in which the surface of the puncture tip and of the zone adjoining same is continuously coloured,
- Figure 2: a magnified representation of a surgical needle according to the invention which has the zone of the puncture tip coloured in annular form and in which the rear section is continuously coloured, and
- Figure 3: a magnified representation of another surface treatment pattern, which is speckled.

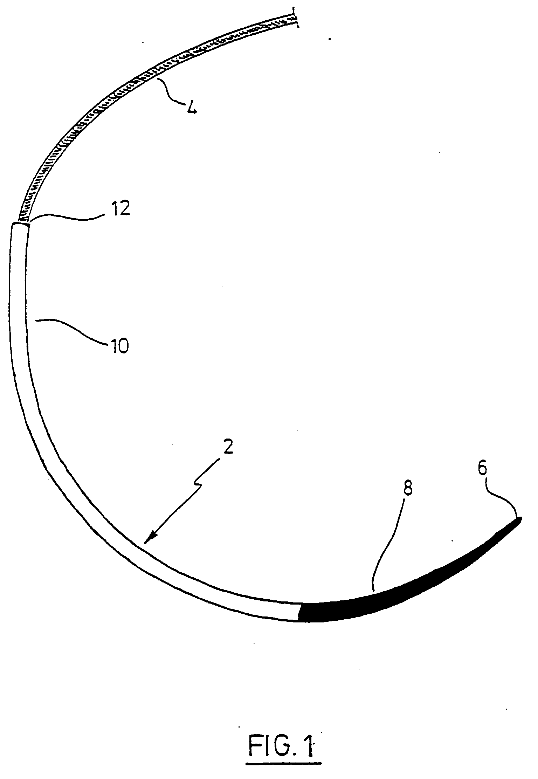

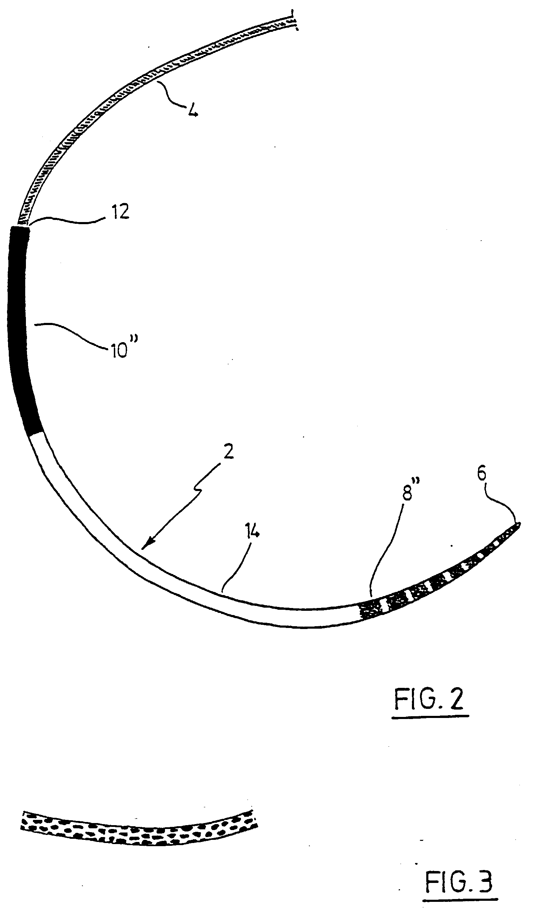

The needle 2 shown in Figures 1 and 2 is a normal semi-circular round-bodied needle; the needle can, however, have any desired form and be, for example, a blunt round-bodied needle, a cutting needle or a spatula needle. Located at one end of the needle 2 is the puncture tip 6, which can also be designed as a microtip, while a thread attachment 12, where a thread 4 made from surgical suture material is secured, is formed at the opposite end of the needle 2.

In the version according to Figure 1, the surface of the puncture tip 6 and of the zone 8 of the needle 2 adjoining same is continuously coloured by chemical or electrolytic means or by a covering. The surface-treated zone accounts for less than 50% of the needle length and preferably less than 25% of the needle length.

A suitable colouring can be achieved either by chemical means through pickling or etching, or electrolytically through appropriate anodic or cathodic treatment, where appropriate with polarity inversion or through alternating current. A special torn of pickling is so-called dipping or matt-dipping. Stoving lacquers can also be used for colouring.

The surface of the remaining section 10 of the needle 2 is untreated, i.e. consists of bare or untreated metal, up to the thread attachment 12 in the version according to Figure 1.

Figure 2 shows a version in which the surface of the puncture tip 6 and that of the zone 8'' of the needle 2 adjoining same is coloured in annular form, whereby the coloured rings, which can be of any width, are ca. 1 to 2 mm apart. The rear section 10'' of the needle 2 is continuously coloured. In between lies the middle zone 14, whose surface consists of bare or untreated metal and which preferably extends over at least 50% of the needle length.

Figure 3 shows another modification of the surface treatment pattern, in which the surface is coloured in the form of speckles. The gaps between the speckles are not to exceed 1 to 2 mm.

With all the needles shown, the colour in the coloured zones can agree with the colour of the thread 4, which offers the additional advantage that the coding colour of the thread is intuitively detectable when needles are in packs.

## Claims

1. Surgical needle with a suture material or thread (4) secured to the end opposite the puncture tip (6), wherein the surface of the puncture tip (6) and of the zone (8) of the needle (2) adjoining same is surface-treated, while the surface of the remaining section (10) of the needle up to the thread attachment (12) consists of bare or untreated metal, characterized in that the surface of the puncture tip (6) and of the zone (8) of the needle (2) adjoining same is coloured, continuously or with small breaks, by chemical or electrolytic means or by a covering, and without being photoluminescent, for up to ca. 50% of the needle length.

2. Surgical needle with a suture material or thread (4) secured to the end opposite the puncture tip (6), wherein the surface of the puncture tip (6) and of the zone (8'') of the needle (2) adjoining same is surface-treated, characterized in that the surface of the puncture tip (6) and of the zone (8'') of the needle (2) adjoining same up to ca. 50% of the needle length as well as the surface of the rear section (10'') of the needle (2) which can account for up to ca. 50% of the needle length, up to the thread attachment (12), is coloured, continuously or with small breaks, by chemical or electrolytic means or by a covering, and without being photoluminescent, while in the middle section (14) of the needle (2) the surface consists of bare or untreated metal.

3. Surgical needle according to claim 1, characterized in that the zone (8) of the needle (2) whose surface is coloured, continuously or with small breaks, by chemical or electrolytic means or by a covering, and without being photoluminescent, accounts for less than 25% of the needle length.

4. Surgical needle according to claim 2, characterized in that the middle section (14) of the needle (2) whose surface consists of bare or untreated metal accounts for at least 50% of the needle length.

5. Surgical needle according to one of the previous claims, characterized in that the coloured zone or zones of the needle are coloured in annular form (8'') or in speckled form, the surface between the rings or speckles being bare or untreated.

## Patentansprüche

1. Chirurgische Nadel mit einem an dem der Einstichspitze (6) entgegengesetzten Ende befestigten Nahtmaterial oder Faden (4), wobei die Oberfläche der Einstichspitze (6) und des sich daran anschließenden Bereichs (8) der Nadel (2) oberflächenbehandelt ist, während die Oberfläche des restlichen Teils (10) der Nadel bis zuin Fadenansatz (12) aus blankem oder unbehandeltem Metall besteht, dadurch gekennzeichnet, daß die Oberfläche der Einstichspitze (6) und des sich daran anschließenden Bereichs (8) der Nadel (2) über bis zu ca. 50% der Nadellänge chemisch oder elektrolytisch oder durch einen Überzug durchgehend oder mit geringfügigen Unterbrechungen gefärbt ist, ohne photolumineszent zu sein.

2. Chirurgische Nadel mit einem an dem der Einstichspitze (6) entgegengesetzten Ende befestigten Nahtmaterial oder Faden (4), wobei die Oberfläche der Einstichspitze (6) und des sich daran anschließenden Bereichs (8'') der Nadel (2) oberflächenbehandelt ist, dadurch gekennzeichnet, daß die Oberfläche der Einstichspitze (6) und des sich daran anschließenden Bereichs (8'') der Nadel (2) über bis zu ca. 50% der Nadellänge sowie die Oberfläche des hinteren Teils (10'') der Nadel (2), der bis zu ca. 50% der Nadellänge ausmachen kann, bis zuin Fadenansatz (12) chemisch oder elektrolytisch oder durch einen Überzug durchgehend oder mit geringfügigen Unterbrechungen gefärbt ist, ohne photolumineszent zu sein, während im mittleren Bereich (14) der Nadel (2) die Oberfläche aus blankem oder unbehandeltem Metall besteht.

3. Chirurgische Nadel nach Anspruch 1, dadurch gekennzeichnet, daß der Bereich (8) der Nadel (2), dessen Oberfläche chemisch oder elektrolytisch oder durch einen Überzug durchgehend oder mit geringfügigen Unterbrechungen gefärbt ist, ohne photolumineszent zu sein, weniger als 25% der Nadellänge ausmacht.

4. Chirurgische Nadel nach Anspruch 2, dadurch gekennzeichnet, daß der mittlere Bereich (14) der Nadel (2), dessen Oberfläche aus blankem oder unbehandeltem Metall besteht, mindestens 50% der Nadellänge ausmacht.

5. Chirurgische Nadel nach einem der vorhergehenden Ansprüche, dadurch gekennzeichnet, daß der bzw. die gefärbten Bereiche der Nadel in Ringform (8'') oder in Sprenkelform gefärbt sind, wobei die Oberfläche zwischen den Ringen oder Sprenkeln blank oder unbehandelt ist.

## Revendications

1. Aiguille chirurgicale ayant un matériau ou fil de suture (4) fixé sur l'extrémité opposée à la pointe de piqûre (6), dans laquelle la surface de la pointe de piqûre (6) et d'une zone (8) de l'aiguille (2) adjacente à celle-ci est traitée en surface, alors que la surface du tronçon restant (10) de l'aiguille allant jusqu'à la fixation du fil (12) est constituée d'un métal nu ou non-traité, caractérisée en ce que la surface de la pointe de piqûre (6) et de la zone (8) de l'aiguille (2) adjacente à celle-ci est colorée, en continu ou en ayant de petites interruptions, par des moyens chimiques ou électrolytiques ou par un revêtement, et sans être photoluminescente, allant jusqu'à environ 50 % de la longueur de l'aiguille.

2. Aiguille chirurgicale ayant un matériau ou fil de suture (4) fixé à l'extrémité opposé à la pointe de piqûre (6), dans laquelle la surface de la pointe de piqûre (6) et de la zone (8'') de l'aiguille (2) adjacente à celle-ci est traitée en surface, caractérisée en ce que la surface de la pointe de piqûre (6) et de la zone (8'') de l'aiguille (2) adjacente à celle-ci constitue jusqu'à environ 50 % de la longueur de l'aiguille ainsi que la surface du tronçon arrière (10'') de l'aiguille (2) qui peut être prise en compte pour constituer les 50 % de la longueur d'aiguille, allant jusqu'à la fixation du fil (12), est colorée, en continu ou en ayant de petites interruptions, par des moyens chimiques ou électrolytiques ou par un revêtement, et sans être photoluminescentes, alors que dans le tronçon médian (14) de l'aiguille (2) la surface est constituée de métal nu ou non-traité.

3. Aiguille chirurgicale selon la revendication 1, caractérisée en ce que la zone (8) de l'aiguille (2) dont la surface est colorée, en continu ou en ayant de petites interruptions, par des moyens chimiques ou électrolytiques, ou par un revêtement, et sans être photoluminescente, constitue moins de 25 % de la longueur de l'aiguille.

4. Aiguille chirurgicale selon la revendication 2, caractérisée en ce que le tronçon médian (14) de l'aiguille (2) dont la surface est constituée d'un métal nu ou non-traité constitue au moins 50 % de la longueur de l'aiguille.

5. Aiguille chirurgicale selon l'une quelconque des revendications précédentes, caractérisée en ce que la zone ou les zones colorées de l'aiguille sont colorées selon une forme annulaire (8'') ou selon une forme mouchetée, la surface entre les anneaux ou les mouchetures étant nue ou non-traitée.
